# EUROPEAN PATENT APPLICATION

(11) **EP 2 095 794 A1**
(43) Date of publication of application: **02.09.2009**
(21) Application number: 09153361.2
(22) Date of filing: 20.02.2009
(51) Int. Cl.: A61F 2/36, A61F 2/30

(54) **Prosthesis**

(30) Priority: 28.02.2008 GB 0803679
(71) Applicant: Finsbury (Development) Limited, Leatherhead Surrey KT22 7BA (GB)
(72) Inventor: Tuke, Michael Anthony, Guildford, Sussex GU1 3TF (GB); Taylor, Andrew Clive, Chichester, Sussex PO19 3QQ (GB)
(74) Representative: Smaggasgale, Gillian Helen

(57) **Abstract**

A prosthesis (21) having an outer articulating surface (22) and an inner bone-contacting surface (23), wherein the inner surface comprises at least one osteo-integration zone (24a,24b) comprising one or more surface formations (25) integrally cast with the wall of the prosthesis.

## Description

The present invention relates to a prosthesis. More particularly, it relates to a prosthesis for use in femoral head resurfacing and a process for the manufacture thereof.

The efficient functioning of the hip joints is extremely important to the well being and mobility of the human body. Each hip joint is comprised by the upper portion of the femur which terminates in an offset bony neck surmounted by a ball-headed portion, the femoral head, which rotates within a socket, known as the acetabulum, in the pelvis. Diseases such as rheumatoid- and osteo-arthritis can cause erosion of the cartilage lining of the acetabulum so that the ball of the femur and the hip bone rub together causing pain and further erosion. Bone erosion may cause the bones themselves to attempt to compensate for the erosion which may result in the bone being reshaped. This misshapen joint may cause pain and may eventually cease to function altogether.

Operations to replace the hip joint with an artificial implant are well-known and widely practiced. Generally, the hip prosthesis will be formed of two components, namely: an acetabular component which lines the acetabulum and a femoral head component, which may be known as a stem component which replaces the femoral head. During the surgical procedure for implanting the femoral head component, the bone femoral head will be removed and the femur hollowed using reamers and rasps to accept the prosthesis. The femoral component will then be inserted into the femur.

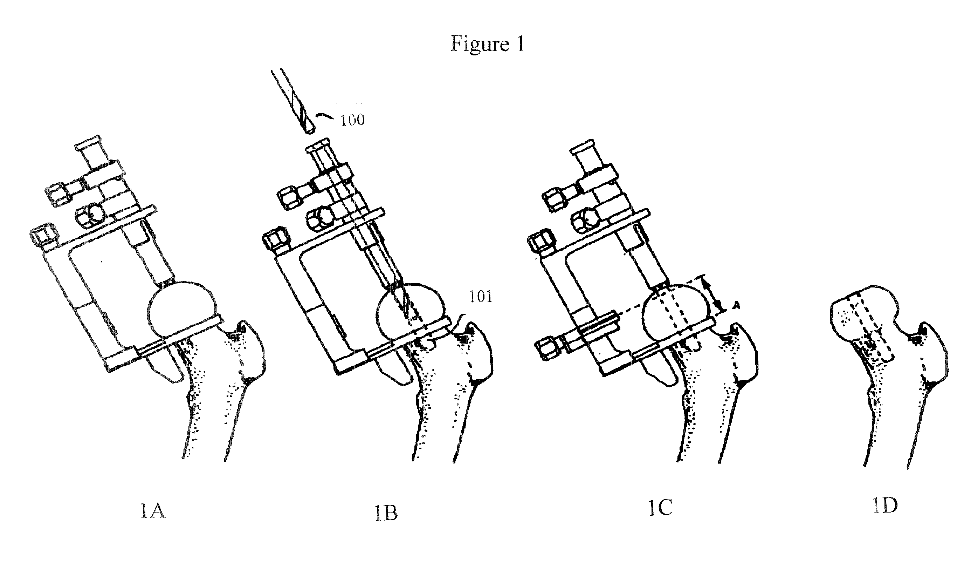

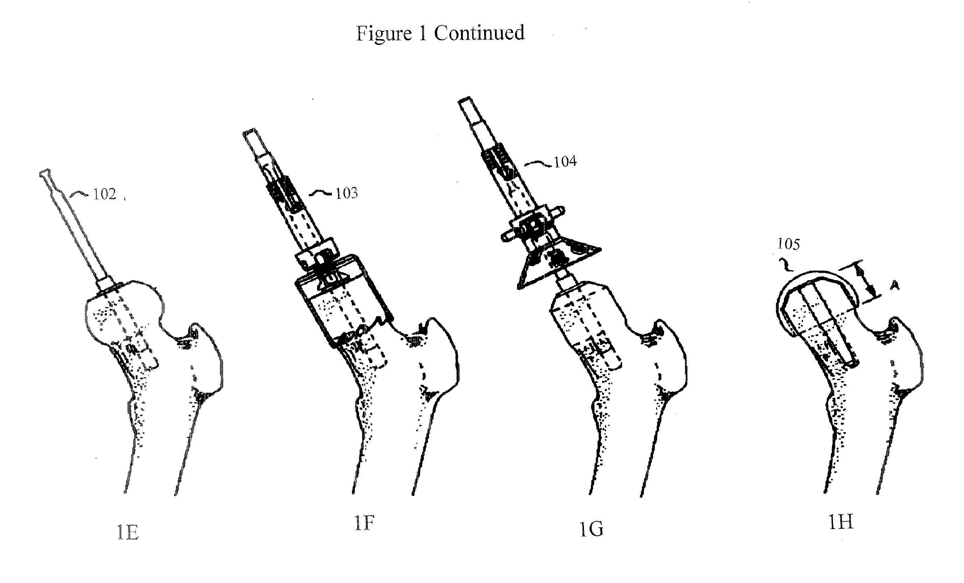

More recently in order to reduce the amount of bone which has to be removed, the technique of femoral head resurfacing has become more widely used and, where appropriate, is replacing procedures in which stem protheses are used. In femoral head resurfacing, the ball head of the femur is machined so that a replacement metal surface can be provided on the machined head. In a conventional resurfacing procedure, the dome of the femoral head is initially removed, usually with a saw. A well is drilled into the head of the femur either before or after the dome is removed and cylinder cutters are then used to shape the femoral head until it has a cylindrical profile. At least the lower edges of the cylinder may then be chamfered. A schematic diagram of this procedure is illustrated in Figure 1.

In the illustrated example, in an alignment guide is located beneath the head of the femur at the head-neck junction (FIG. 1A). A drill 100 is then inserted into the collar, optionally through the bore of the cannulated rod, and a well 101 is drilled in the head of the femur (FIG. 1B). The top of the head of the femur is then removed by sawing using the saw guide when present to ensure that the cut is the correct distance A from the ring of the alignment guide which represents the skirt of the prosthesis (FIG. 1C). It will be understood that it some arrangements, the top of the femur may be removed before the well is drilled. The alignment guide is then removed (FIG. 1D). A guide rod 102 is then inserted into the well (FIG. 1E). This guide rod is then used to locate and correctly angle a sleeve cutter 103 and a chamfering tool 104 (FIGS. 1F and 1G). The femoral head resurfacing prosthesis 105 can then be applied as illustrated in FIG. 1H. It can be seen that the distance from the cut head of the femur to the edge of the skirt of the prosthesis is A. Alternative guides can be used to correctly angle the well.

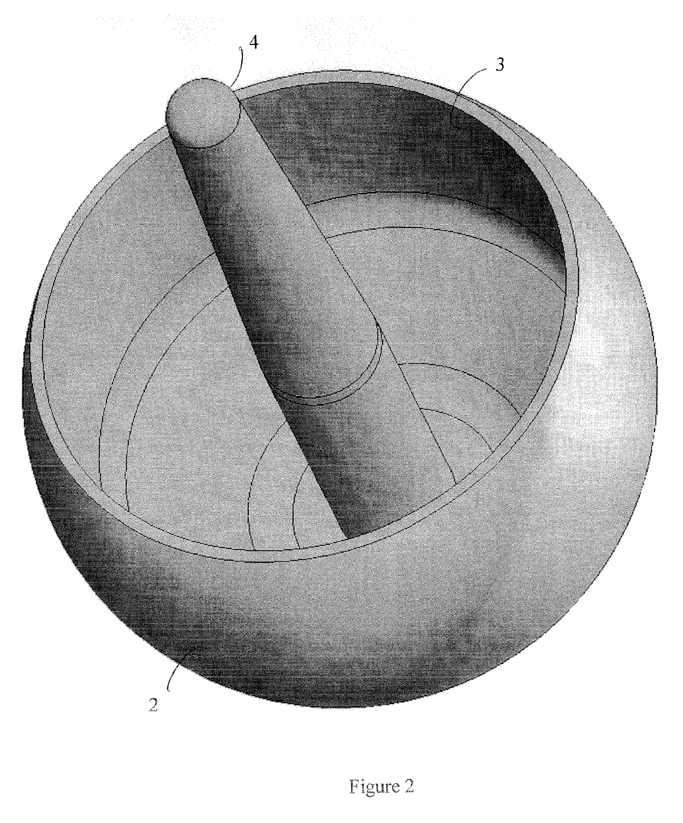

A conventional femoral head resurfacing prosthesis 1 is illustrated in Figure 2. The outer surface 2 of the prosthesis is shaped to mimic an ideal natural femoral head. The inner surface 3 is of cylindrical configuration which will fit over the remains of the natural femur after it has been machined. A support pin 4 is provided. The resurfacing head and the support pin are generally cast in one piece. In use, the support pin is inserted into the well in the machined femoral head to lock the prosthesis in position and to provide stability. These femoral head prosthesis and the operative techniques for their use are well known in the art.

It is important that the well in the head of the machined femur is precisely positioned such that in use, where the support pin 4 is inserted in the well, the load is correctly carried. Errors in the positioning and/or angling of the well can result in post-operative failure of the prosthesis or even damage to the bone such as a fracture occurring. In view of these problems many proposals have been made for guides, jigs and the like which will assist the surgeon to locate the correct position for the well that will be drilled into the head of the machined femur to which the prosthesis is to be applied.

In order to ensure that the femoral head prosthesis is securely anchored in the femur, the diameter of the well in the femur is generally smaller than the diameter of the support pin of the implant. In use, the support pin has to be driven into the well such that it is a tight fit. This is usually achieved using a mallet. The stress to the femur as the pin is driven into the well can cause cracks to occur in the bone which, although are not noticeable during surgery, can propagate post-operatively.

In addition to the anchoring achieved by the presence of the support pin, the femoral head resurfacing prosthesis will generally be secured to the machined femoral head using bone cement.

Bone cement typically comprises finely divided polymer components, such as polymethylmethylacrylate,which are mixed with a liquid monomer such as an acrylic ester. A polymerizing initiator is then added to the mixture which with time causes the mixture to polymerize and harden.

The polymerization process is an exothermic reaction and thus as the bone cement cures there will be an increase in temperature. Prior to the application of the resurfacing head prosthesis to the machined bone, uncured bone cement is located in the inner cavity of the prosthesis. Once the prosthesis is in position and curing commences, there can be a substantial rise in temperature in a fairly localised area. Although the conducting properties of the metal prosthesis can act to dissipate some of the heat, it is possible that at some places, the temperatures reached may be sufficient to cause damage to the bone.

A further disadvantage associated with the use of cement is that when the prosthesis is applied to the machined bone, the cement which has been applied into the inner surface of the prosthesis can exit from the space between the bone and the prosthesis as it is located in position on the machined femoral head and run down the femur. It is therefore necessary for precautions to be taken to ensure that the cement does not enter the wound.

It is also desirable to avoid the use of bone cement since there is a greater opportunity for the implant to form a bond with the bone. Whilst proposals have been made to develop prosthetic implants having surface finishes which are intended to promote bone ingrowth and bonding to the implant. In one arrangement, the bone-contacting portions of many prosthetic implants is provided with a surface consisting of hydroxyapetite which promotes bone ingrowth.

It has now been discovered that if the inner surface of the resurfacing prosthesis is coated with osteo-integration formations, not only is it possible to avoid the use of bone cement, it may also be possible to do without the support pin therefore obviating the need to drill a well into the femoral head. The absence of the well not only avoids weakening the centre of the femoral head but also removes the problems which arise if the well is not drilled at the optimum angle.

With a view to addressing this problem, it has been suggested that the inner surface of the femoral head resurfacing prothesis should have beads of an appropriate size bonded to the internal surface thereof. These beads are applied to the pre-formed prosthesis in a heat sintering process. Whilst this arrangement goes some way to addressing the problems associated with the use of bone cement, it still suffers from various disadvantages and drawbacks.

For example, the heat process used to apply the beads by sintering effects the metallurgy of the prosthesis such that the beads are very friably loose meaning that they can become detached. If they are detached while the prosthesis is being attached to the machined femoral head, they may enter the body causing post-operative problems. If they become detached once the femoral head is in position such that they are retained between the machined femoral head and the prosthesis, they can cause wear to the prosthesis and/or damage to the bone.

In addition, the manufacturing method of the prior art arrangement makes it difficult to control the positioning of the beads which can lead to a densely packed surface which may actually inhibit osteo-integration.

The present invention therefore relates to an improved femoral head resurfacing prosthesis and a process for the manufacture thereof.

Thus according to the present invention there is provided a prosthesis having an outer articulating surface and an inner bone-contacting surface, wherein the inner surface comprises at least one osteo-integration zone comprising one or more surface formations integrally cast with the wall of the prosthesis.

Although the present invention will be described with particular reference to a femoral head prosthesis, it will be understood that the invention is equally applicable to other prostheses which may be used to resurface the articulating surface of a bone

Any suitably shaped formations may be used provided that they facilitate the desired osteo-integration.

In one arrangement the shaped formations may be geometrical projections from the inner surface of the prosthesis and may be cuboid, rhomboid, pyramidal and the like. In one arrangement the shaped formations may be of the configuration described in EP1527757 which is incorporated herein by reference.

In a preferred arrangement, the shaped formations are portions of spheres. The portions are generally at least hemispherical but may be larger.

As the shaped formations are cast with the prosthesis they are an integral component therewith. This gives superior advantages as far as strength and integrity are concerned.

The osteo-integration zone may cover the entirety of the inner bone-contacting surface or of selected areas thereof. Since the structure is formed by casting, the precise location of the zone can be carefully controlled.

The surface formations may be of any suitable size. Suitable sizes for the part-spherical formations include those having diameters from about 0.5mm to about 1.5mm, more preferably from about 0.9mm to about 1.1mm.

The surface formations may be located at any suitable spacing. For enhanced osteo-integration, spacings of the order of from about 0.2mm to about 0.8mm may be used with spacings of about 0.4mm offering particular advantages.

In one embodiment, the osteo-integration zone may be at least partly coated with an osteo-genesis inducing agent. Any agent which promotes bone growth may be used, although hydroxyapatite is particularly preferred.

In some arrangements of the prosthesis of the present invention, the osteo-integration which occurs due to the presence of the osteo-integration zone is sufficient to provide the desired attachment such that the conventional support pin can be omitted from the prosthesis. Thus, in a preferred embodiment of the present invention, the prosthesis does not include a support pin. The absence of the support pin allows the prosthesis to be implanted without having to drive the support pin into a well in the femur. Additionally, if the prosthesis is provided without a support pin, the problems associated with correctly orientating the well in the femoral head is obviated.

According to a second aspect of the present invention there is provided a process for the manufacture of the prosthesis of the above first aspect comprising the steps of:
preparing a mould; and
casting the prosthesis including the surface formations.

The present invention will now be described by way of example according to the accompanying figures in which:
- Figure 1: is a schematic illustration of the steps requires to machine a femoral head for resurfacing with a conventional head prosthesis;
- Figure 2: is a perspective view of a conventional resurfacing prosthesis; and
- Figure 3: is a perspective view of one embodiment of the present invention.

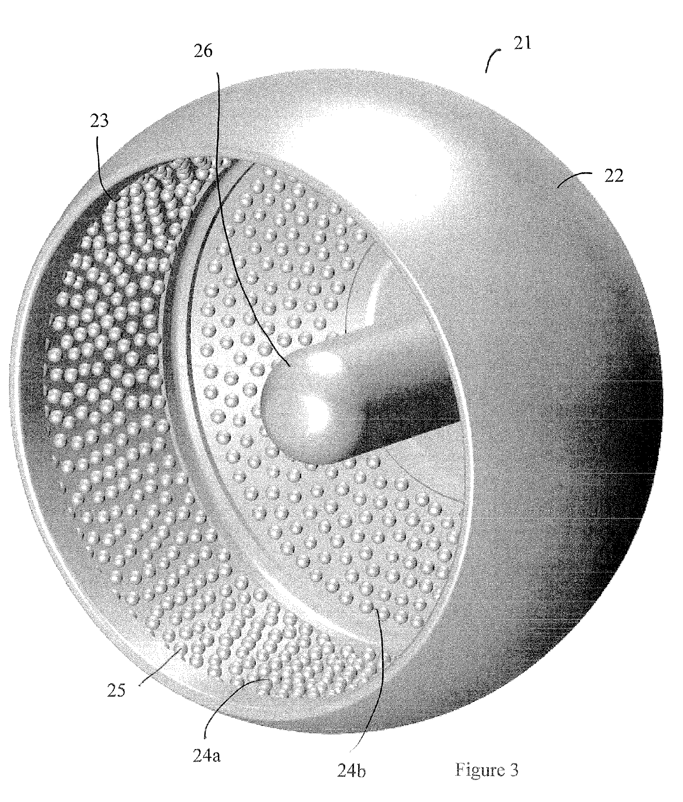

The prosthesis 21 of the present invention is illustrated in Figure 3. It comprises an outer surface 22 and an inner surface 23. Two osteo-integration zones 24a and 24b are located on the inner surface. Part-spherical surface formations 25 are located in the osteo-integration zones 24a and 24b. In the illustrated embodiment a support pin 26 is present although it may be omitted.

In use the surgeon will machine the femoral head as for conventional resurfacing techniques although where the support pin is not present in the prosthesis, the well will not be drilled into the head. The prosthesis of the present invention can be located in position on the machined head without the need for cement to be used.

## Claims

1. A prosthesis having an outer articulating surface and an inner bone-contacting surface, wherein the inner surface comprises at least one osteo-integration zone comprising one or more surface formations integrally cast with the wall of the prosthesis.

2. A prosthesis according to Claim 1 wherein the prosthesis is a femoral head prosthesis.

3. A prosthesis according to Claim 1 or 2 wherein the shaped formations are geometrical projections.

4. A prosthesis according to Claim 1 or 2 wherein the shaped formations are portions of spheres.

5. A prosthesis according to Claim 4 wherein the shaped formations are at least hemispherical.

6. A prosthesis according to any one of Claims 1 to 5 wherein the osteo-integration zone covers the entirety of the inner bone-contacting surface.

7. A prosthesis according to any one of Claims 1 to 5 wherein the osteo-integration zone covers a selected area of the inner bone-contacting surface.

8. A prosthesis according to Claim 4, wherein the part-spherical formations have diameters of from about 0.5mm to about 1.5mm.

9. A prosthesis according to any one of Claims 1 to 8 wherein the surface formations are located at spacings of the order of from about 0.2mm to about 0.8mm.

10. A prosthesis according to any one of Claims 1 to 9 wherein the osteo-integration zone is at least partly coated with an osteo-genesis inducing agent.

11. A process for the manufacture of the prosthesis of any one of Claims 1 to 10 comprising the steps of:
preparing a mould; and
casting the prosthesis including the surface formations.
